# EUROPEAN PATENT SPECIFICATION

(11) **EP 1 200 445 B1**
(45) Date of publication and mention of the grant of the patent: **17.09.2003**
(21) Application number: 00958313.9
(22) Date of filing: 28.07.2000
(51) Int. Cl.: C07D 495/04, A61K 31/496, A61P 9/00

(54) **THIENOPYRANECARBOXAMIDE DERIVATIVES**
THIENOPYRAN-CARBOXAMID-DERIVATE
DERIVES DE THIENOPYRANECARBOXAMIDE

(30) Priority: 30.07.1999 IT MI991704
(43) Date of publication of application: 02.05.2002
(73) Proprietor: RECORDATI INDUSTRIA CHIMICA E FARMACEUTICA S.p.a., 20148 Milano (IT); RECORDATI S.A. CHEMICAL and PHARMACEUTICAL COMPANY, 6830 Chiasso (CH)
(72) Inventor: LEONARDI, Amedeo, I-20154 Milano (IT); MOTTA, Gianni, I-20030 Barlassina (IT); RIVA, Carlo, I-21100 Varese (IT); TESTA, Rodolfo, I-20060 Vignate (IT)
(74) Representative: McKelvey, Ian Edward
(86) International application number: EP0007306
(87) International publication number: WO01009140

(56) References cited:
- EP-A- 0 558 245
- WO-A-99/06382
- WO-A-99/06384

## Description

The invention relates to thienopyranecarboxamide derivatives, to pharmaceutical compositions containing them and to uses for such derivatives and compositions.

### BACKGROUND OF THE INVENTION

US 5403842 and its continuations in part (US 5474994 and 5605896) claim heterobicyclic derivatives bearing substituted phenylpiperazines as basic moieties linked to the heterocyclic ring by a variety of spacer groups. Among said derivatives, compound A (Example 11, Rec 15/2739) is of relevant interest due its very high uroselective activity. Compound A, in fact, is endowed with good affinity for the α_{1A} adrenoceptor and is able to selectively inhibit contractility of the prostatic urethra in a dog model without substantial effect on blood pressure (Leonardi A. et al., *J. Pharmacol. Exp. Therap.* 281, 1272-1283 (1997).

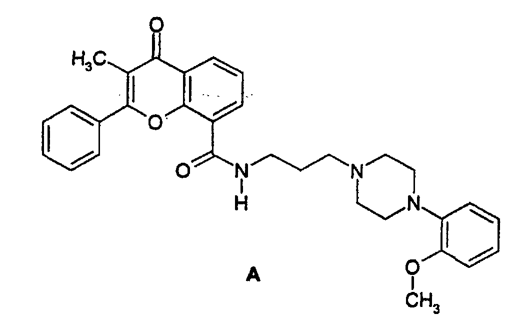

7-Oxo-7H-thieno[3,2-*b*]pyran-3-carboxylic acid and its *N*,ω-aminoalkylamides are compounds not yet reported in the literature. This invention is directed to the new structural class of the *N*-substituted phenyl-*N*',ω-(5-substituted-7-oxo-7*H*-thieno[3,2-*b*]pyran-3-carbonylamino)-alkylpiperazines.

Compounds of this class are endowed with enhanced selectivity toward the α₁ adrenergic receptor and improved *in vivo* uroselectivity with regard, for example, to compound A, with remarkable effects on relaxation of prostatic urethra and very low activity in lowering blood pressure. This activity profile suggests the safe use of the compounds of the invention in the therapy of obstructive syndromes of the lower urinary tract including benign prostatic hyperplasia (BPH), in the therapy of lower urinary tract symptoms (LUTS) and in the therapy of neurogenic lower urinary tract dysfunction (NLUTD), all without side effects associated with hypotensive activity.

### SUMMARY OF THE INVENTION

In one aspect, the invention is directed to compounds of Formula I: 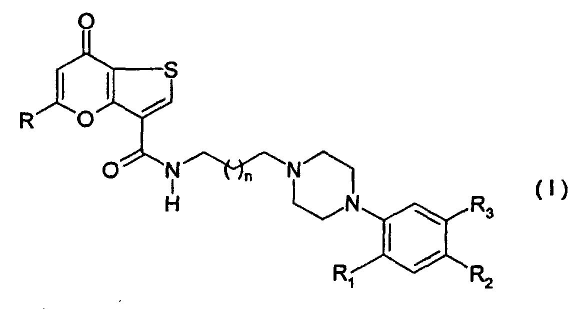 wherein
R is an aryl, cycloalkyl or polyhaloalkyl group,
R₁ is an alkyl, alkoxy, polyfluoroalkoxy, hydroxy or trifluoromethanesulphonyloxy group,
each of R₂ and R₃ independently represents a hydrogen or halogen atom or an alkoxy or polyfluoroalkoxy group, and
n is 0, 1 or 2.

The preferred aryl group R is phenyl, the preferred cycloalkyl group R is cyclohexyl and the preferred polyhaloalkyl group R is trifluoromethyl. The preferred alkyl groups R₁ are lower alkyl groups having from 1 to 4 carbon atoms, in particular methyl, the preferred alkoxy groups R₁ are lower alkoxy groups, in particular methoxy; the preferred polyfluoroalkoxy groups R₁ are trifluoromethoxy or 2,2,2-trifluoroethoxy groups. R₂ is preferably a hydrogen or fluorine atom and R₃ is preferably a hydrogen or chlorine atom or a 2,2,2-trifluoroethoxy group. The preferred value for n is 1.

The invention also includes the N-oxides and pharmaceutically acceptable salts of these compounds.

The invention further provides pharmaceutical compositions comprising a compound of Formula I or an N-oxide or pharmaceutically acceptable salt of such a compound in admixture with a pharmaceutically acceptable diluent or carrier. Such pharmaceutical compositions may optionally further comprise an anticholinergic agent, for example one or more of tolterodine, oxybutinin, darifenacin, alvameline and temiverine.

### DETAILED DESCRIPTION OF THE INVENTION

All patents, patent applications and literature references cited in this application are incorporated by reference in their entirety.

The adrenergic antagonistic activity of the compounds of the invention renders them useful as agents acting on body tissues particularly rich in α₁-adrenergic receptors such as prostate and urethra. Accordingly, the anti-adrenergic compounds within the invention, established as such on the basis of their receptor binding profile, can be useful therapeutic agents for the treatment, for example, of micturition problems associated with obstructive disorders of the lower urinary tract, including but not limited to benign prostatic hypertrophy (BPH).

BPH is a progressive condition, which is characterised by a nodular enlargement of prostatic tissue resulting in obstruction of the urethra. This results in increased frequency of urination, nocturia, a poor urinary stream and hesitancy or delay in starting urine flow. Chronic consequences of BPH can include hypertrophy of bladder smooth muscle, a decompensated bladder and an increased incidence of urinary tract infection. The specific biochemical, histological and pharmacological properties of a prostate adenoma leading to the bladder outlet obstruction are not yet known. However, the development of BPH is considered to be an inescapable phenomenon for the ageing male population. BPH is observed in approximately 70% of males over the age of 70. Currently, the worldwide stated method of choice for treating BPH is surgery. A medicinal alternative to surgery is clearly very desirable. The limitations of surgery for treating BPH include the morbidity rate of an operative procedure in elderly men, persistence or recurrence of obstructive and irritative symptoms, as well as the high cost of surgery.

α-Adrenergic receptors (McGrath et al., *Med. Res. Rev.* 9, 407-533 (1989)) are specific neuroreceptor proteins located in the peripheral and central nervous systems on tissues and organs throughout the body. These receptors are important switches for controlling many physiological functions and, thus, represent important targets for drug development. In fact, many α-adrenergic drugs have been developed over the past 40 years. Examples include clonidine, phenoxybenzamine and prazosin, terazosin, alfuzosin, doxazosin, tamsulosin (treatment of hypertension), naphazoline (nasal decongestant), and apraclonidine (treating glaucoma). α-Adrenergic drugs can be broken down into two distinct classes: agonists (clonidine and naphazoline are agonists), which mimic the receptor activation properties of the endogenous neurotransmitter norepinephrine, and antagonists (phenoxybenzamine and prazosin, terazosin, alfuzosin, doxazosin, tamsulosin are antagonists), which act to block the effects of norepinephrine. Many of these drugs are effective, but also produce unwanted side effects (for example, clonidine produces dry mouth and sedation in addition to its antihypertensive effects). The above reported agonists are selective for the α₂ adrenergic receptor whereas most antagonists are selective for the α₁ adrenoceptor, with the exception of tamsulosin which shows a relevant affinity also for the 5-HT_{1A} receptor. Many of the cited α₁ antagonists are currently used for the therapy of BPH but, due to their poor uroselectivity, they are liable to cause side effects of cardiovascular origin.

Recent pharmacological, biochemical and radioligand-binding studies evidenced three different α₁-receptor subtypes with a high affinity for prazosin, namely α_{1A}- (α₁ₐ-), α_{1B}- (α_{1b}-) and α_{1D}- (α_{1d}-), with lower case subscripts being used for recombinant receptors and upper case subscripts for receptors in native tissues (Hieble et al., Pharmacol. Rev. 47, 267-270, 1995). In functional studies α₁-receptors with a low affinity for prazosin have also been identified and termed α_{1L}-receptors (Flavahan and Vanhoutte, *Trends Pharmacol. Sci.* 7, 347-349, 1986; Muramatsu et al., *Pharmacol. Comm*. 6, 23-28, 1995).

Several studies have demonstrated the presence of these α₁-adrenergic receptor subtypes in the lower urinary tract tissues, as reviewed by K.E. Andersson in the proceedings of the "4^{th} International Consultation in Benign Prostatic Hyperplasia (BPH)" held in Paris, July 2-5, 1997 (pages 601-609).

Several studies have shown that the human prostate receives innervation from both the sympathetic and parasympathetic nervous systems.

The adrenergic nerves are considered responsible for prostatic smooth muscle tone by releasing noradrenaline, stimulating contraction-mediating α adrenoceptors. Approximately 50% of the total urethral pressure in BPH patients may be due to α-adrenoceptor-mediated muscle tone. Functional studies have indicated the occurrence of important adrenoceptor functions in prostatic adenomatous and capsular tissue. Clinical studies with the prototypical adrenoceptor-selective antagonist, prazosin, reinforced the key role of α₁ adrenoceptors in the control of prostatic smooth-muscle tone. This was also confirmed in the laboratory by studies showing that, although both α₁-and α₂-adrenoceptors can be identified within the human prostate, contractile properties are mediated primarily by α₁ adrenoceptors. Many clinical investigations have confirmed that α₁-adrenoceptor blockade relieves lower urinary tract symptoms (LUTS), both of storage (irritative) and voiding (obstructive) type, in patients with BPH.

Lower urinary tract symptoms (LUTS) also develop in women as they age. As in men, LUTS in women includes both filling symptoms such as urgency, incontinence and nocturia, and voiding symptoms, such as weak stream, hesitancy, intermittency, incomplete bladder emptying and abdominal straining. That both men and women experience a similar high prevalence of filling and voiding LUTS suggests that at least part of the underlying etiology may be identical. In a recent study, an α₁-antagonist was reported to reduce LUTS in women to a greater extent than an anticholinergic (Serels, S. and Stein, M., *Neurology and Urodynamics* 17:31-36, 1998). The authors concluded that there appeared to be a role for α₁-antagonists in treating LUTS in women. The possible mechanisms implicated to explain these results are: a) dysfunction of the bladder neck and urethra, causing functional outlet obstruction, analogous to BPH-induced outlet obstruction, with secondary detrusor overactivity; and b) increased α₁-adrenoceptor activity in the detrusor, causing frequency and urgency. On these bases, α₁-antagonists are used in clinical practice to treat LUTS in women (Fitzpatrick, *Brit. J*. *Urol. Intl*. 85, Supp.2:1-5,2000; Kakizaki, M. et al., *Brit. J*. *Urol. Intl*. 85, Supp.2:25-30, 2000). The results of Serels also indicate that the combined administration of α₁-antagonists and anticholinergics can have improved efficacy in treatment of LUTS, as suggested by Fitzpatrick, *Brit. J. Urol. Intl.* 85, Supp.2:1-5,2000.

Another possible use of α₁-antagonists is the management of neurological lower urinary tract dysfunction (NLUTD), as can be caused by neurological disease or trauma. NLUTD may lead to debilitating symptoms and serious complications, including increased urinary frequency, incontinence, voiding difficulty, recurrent upper urinary tract infections and upper urinary tract deterioration. Management of NLUTD is indicated to preserve renal function and avoid urological complications. Administration of α₁-antagonists may benefit patients with NLUTD by facilitating urine storage by alleviating high detrusor pressure during bladder filling, which is evidenced by poor bladder compliance and detrusor hyperreflexia. In both animal models and patients with spinal cord injury resistant to anticholinergics, α₁-antagonists improved compliance (Kakizaki, M. et al., *Brit. J. Urol. Intl*. 85, Supp.2:25-30, 2000; Sundin, T. et al., *Invest Urol*. 14:322-328, 1977; McGuire et *al., Neurology and Urodynamics* 4:139-142, 1985; Swrerzewski, S.J. et al., *J. Urol.* 151:951-954, 1994).

Two distinct α₁-adrenoceptor subtypes have been suggested to be present in the human prostate, one with high (α_{1H}) and one with low (α_{1L}) affinity for prazosin. All three high-affinity α₁-adrenoceptor subtypes found in molecular cloning studies have been identified in prostatic stromal tissue. The α₁ₐ subtype was found to be the dominant, representing about 60-85% of the α₁-adrenoceptor population. Recent findings suggest that there may be differences in subtype populations between normal and hyperplastic prostates, the ratios between the subtypes α₁ₐ:α_{1b}:α_{1d} being 85:1:14 in BPH and 63:6:31 in non-BPH tissue.

The α_{1A}-adrenoceptor was reported to mediate the contractile response of the human prostate *in vitro.* Ford et al. found that the α_{1A} adrenoceptor may not mediate contractile responses to noradrenaline, and suggested as a candidate the α_{1L} adrenoceptor. Findings by Kenny et al. (*Br. J. Pharmacol.* 118, 871-878 (1996)) support the view that the α_{1L} adrenoceptor, which appears to share many of the characteristics of an α_{1A} adrenoceptor, mediates the contractile response of the human prostate.

In the female urethra, mRNA for the α₁ subtype was predominant and autoradiography confirmed the predominance of the α_{1A} adrenoceptor (Andersson, K.E., *Brit. J. Urol. Intl.* 85, Supp.2:12-18, 2000). The α_{1A} and α_{1D} subtypes are reported to be present in the human detrusor, with the latter subtype predominant (Malloy, B. et al., *J. Urol.* 160:937-943, 1998). Accordingly, the evidence that α₁ adrenoceptor antagonists are useful in treating lower urinary tract symptoms of both prostatic and non-prostatic origin in both males and females can be used to support the usefulness of the compounds of the invention in treating such symptoms regardless of whether they are of obstructive character or not and regardless of the gender of the patient.

On the other hand, it has also been suggested that the α_{1A} and α_{1L} adrenoceptors may represent distinct pharmacological sites of the same receptor.

The affinity of the compounds of the invention for each receptor can be assessed by receptor binding assays, for example as follows:
1) α₁-adrenergic-receptor subtypes: using the specific ligand ³H-prazosin, according to Testa et al., *Pharmacol. Comm.* 6, 79-86, 1995;
2) 5HT_{1A} serotoninergic receptors: using the specific ligand ³H-8-OH-DPAT according to Fargin et al., *Nature* 335, 358-360, 1988.

The α_{1L} adrenergic receptor is not yet cloned and, therefore, the functional affinity of the compounds of the invention for this subtype can be assessed by using an isolated organ preparation as reported by Testa et al., *J*. *Pharmacol. Exp. Ther.* 281, 1284-1293, 1997.

*In vitro* testing of compounds of this invention on the above receptors is described in Examples 8 and 9.

The drugs having α₁-adrenergic antagonistic activity currently used for the symptomatic therapy of BPH are poorly subtype-selective and subject to cause relevant side effects due to their hypotensive activity. Thus there is still a need for selective α₁-antagonists which do not subject the BPH patient to the side effects of said treatments, notably of the cardiovascular type.

The very high uroselectivity of the compounds of this invention has been tested in the dog model described in Example 10, where their efficacy in antagonising the contractions of prostatic urethra in the presence of very limited effects on blood pressure has been shown in comparison with Compound A and another well know α₁-antagonist, prazosin.

Accordingly, it will be understood that the compounds of the invention will be useful I the treatment of BPH while avoiding any undue side effects due to acute hypotension.

It is another object of the present invention to provide pharmaceutical compositions comprising 7-oxo-7H-thieno[3,2-*b*]pyran-3-carboxamido derivatives which are selective α₁-adrenoceptor antagonists, which compositions are effective for the treatment of BPH and other diseases of the lower urinary tract such as LUTS and NLUTD.

The new compounds are also thought to be useful for lowering intraocular pressure and in the treatment of cardiac arrhythmia and erection and sexual dysfunction. Other features and advantages of the present invention will be apparent to those skilled in the art from the following detailed description and appended claims.

### SYNTHESIS OF THE COMPOUNDS OF THE INVENTION

The compounds according to the invention may generally be prepared as follows:

Direct condensation of acids **1** with the ω-aminoalkylammo derivatives **2** (SCHEME 1) 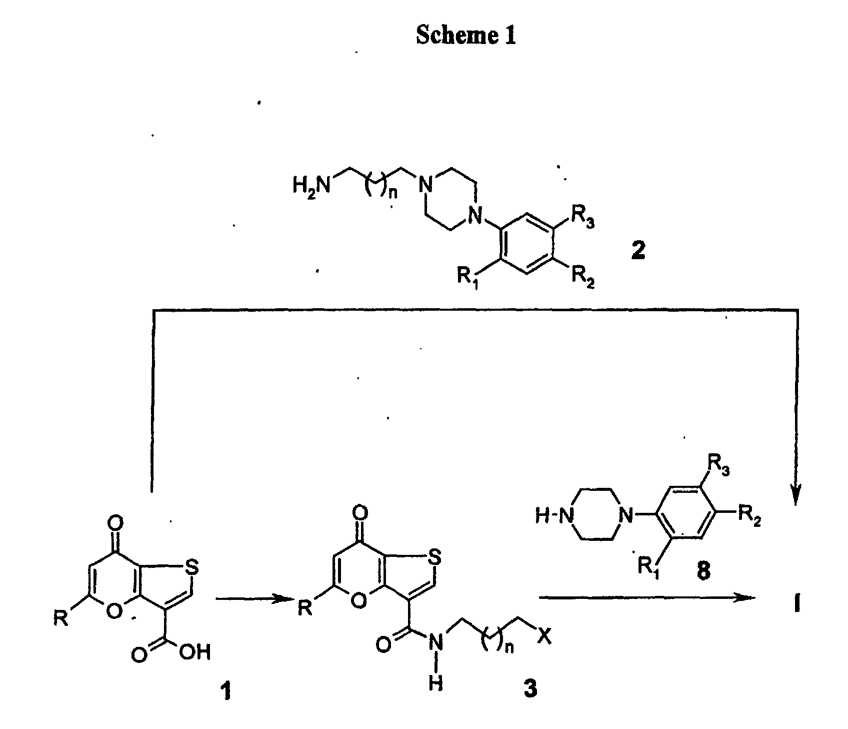 leads to the compounds of the invention. The condensation can be carried out in the presence of a condensing agent (e.g. dicyclohexylcarbodiimide or diethyl cyanophosphonate) optionally in the presence of a promoting agent (e.g. *N*-hydroxysuccinimide, 4-dimethylaminopyridine or *N,N'*-carbonyldiimidazole) in an aprotic or chlorinated solvent (e.g. *N,N*-dimethylformamide or chloroform) at -10/140°C (Albertson, *Org*. *React.* 12, 205-218 (1962); Doherty et al., *J. Med*. *Chem.* 35, 2-14 (1992); Ishihara, *Chem. Pharm. Bull. 39,* 3236 (1991)). In some cases the activated ester or amide intermediates (such as N-hydroxysuccinimidyl esters or acyl imidazolide) can be isolated and further reacted with **2** to be transformed into the corresponding amides (**I**) in an aprotic or chlorinated solvent at 10/100°C. This kind of condensation is well illustrated in the Examples. Another activated intermediate which can be used is the mixed anhydride of **1**, obtainable reacting **1** with an alkyl chloroformate in the presence of a tertiary amine (e.g. triethylamine or *N*-methylmorpholine), which is reacted with **2** at 0-80°C; optionally a promoting agent (e.g. 1-hydroxypiperidine) may be added before the amine addition (Albertson, *Org. React.* 12, 157 (1962)).

Alternatively the condensation can be carried out without a solvent at 150-220°C (Mitchell et al., *J. Am. Chem. Soc.* 53; 1879 (1931)) or in high-boiling ethereal solvents (e.g. diglyme).

Additionally, the condensation can be performed through preparation and optional isolation of reactive derivatives of **1** such as acyl halides. The preparation and use of these last derivatives is well documented in the literature and known to people skilled in the art.

Also less reactive derivatives of **1** can be used, such as alkyl esters, which in turn can be converted into **I** in the presence of a condensing agent (e.g. trimethylaluminum) in an aprotic and/or chlorinated solvent (e.g. hexane, dichloromethane) at -10/80°C, or without solvents at 80-180°C, (S. M. Weinreb et al., *Tetrahedron Lett.* 4171 (1977); M. F. Lipton et al., *Org. Synth*. 59, 49 (1979)).

By the same methods of condensation reported above and using H₂NCH₂(CH₂)ₙCH₂X (with X = halogen or OH) as a reagent, **1** can be transformed into **3**. In the case of X = OH, the conversion of the alcoholic group into the proper leaving group by methods well known to those skilled in the art is then performed. Compounds **3** (with X = halogen or alky/arylsulphonyloxy group) can be subsequently reacted with a phenylpiperazine **8**. The nucleophilic substitution is carried out preferably, but not necessarily, at a temperature within the range of 20-200°C in a polar solvent such as dimethylformamide, acetonitrile, methanol or others, or without any solvent, usually in the presence of a base such as potassium carbonate. See also Gibson's chapter in Patai: "The Chemistry of the Amino Group", p. 45 et seq., Wiley International Science, N.Y., 1968.

The preparation of compounds **2** is disclosed in the literature and is well known to those skilled in the art, and includes nucleophilic substitution of a phenylpiperazine **8** on a *N*-(ω-haloalkyl)phthalimide or a proper ω-haloalkylnitrile or haloalkylamide by the method illustrated above for the condensation of compounds **3** and **8** or by addition of an α,β-unsaturated alkylnitrile or alkylamide in a proper solvent (e.g. acetonitrile, *N,N*-dimethylformamide, a chlorinated solvent or other aprotic polar solvent) at a temperature between 0°C and the reflux temperature of the solvent. A standard phthalimido-group deprotection or reduction of the amido or cyano group then provides compounds **2**.

The acids **1** of the invention in which R represents an alkyl, cycloalkyl or phenyl group can be synthesised (SCHEME 2) starting from methyl 2-acetyl-3-hydroxythiophene-4-carboxylate (prepared as described in J. Chem. Soc. Perkin Trans I, 507 (1986)), which can be esterified with the proper alkanoyl or aroyl chloride by using methods very well known to those skilled in the art. Alternative procedures include the same methods described above for the amidification of **1**, which could be applied as well in the esterification step to afford **4**.

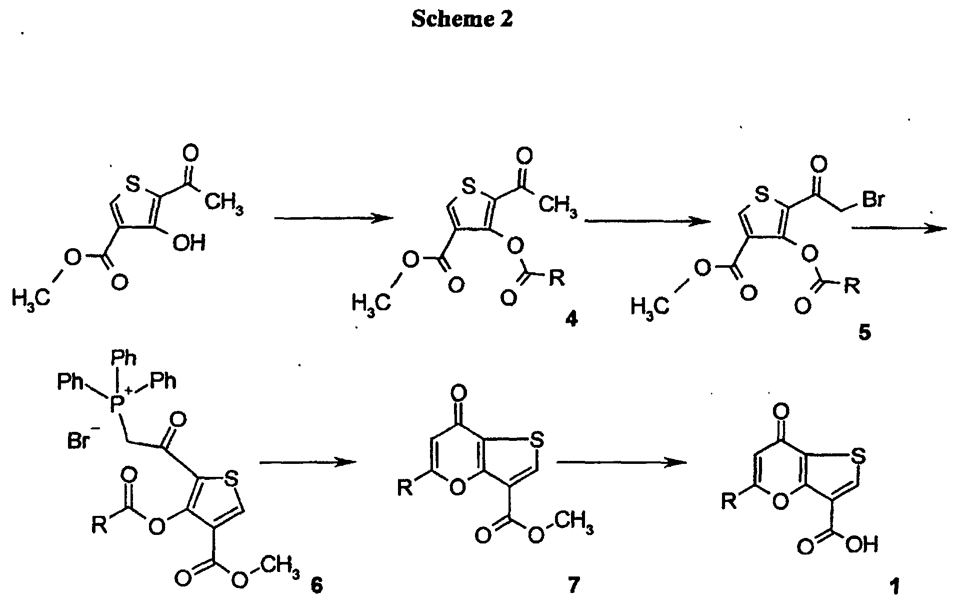

Simple monobromination of the methylketo group of **4** can afford **5**, which can be reacted with triphenylphosphine by usual procedure (acetonitrile or toluene or other aprotic solvent at reflux) to give the phosphonium salt **6**. A subsequent intramolecular ester-Wittig reaction applied to this substrate can afford the thieno[3,2-b]pyranes **7**. Hydrolysis of the ester functionality of **7** by acid or base catalysed procedures well known to those skilled in the art affords compounds **1**.

Very well known hydrolysis procedures include the use of sodium or potassium hydroxide in aqueous ethanol at 40-75°C or lithium hydroxide in aqueous dimethylformamide or dioxane or tetrahydrofuran at 40-100°C.

The compounds **1** where R is a polyfluoroalkyl group can be prepared from 2-acetyl-3-hydroxythiophene-4-carboxylate following the cyclization procedure described by Riva, C. et al., *Synthesis,* 195-201 (1997) by direct cyclization in the presence of anhydrous polyfluoroalkanoyl anhydrides catalysed by 1,8-diazabicycloundec-7-ene.

The compounds **I** where R₁ is a trifluoromethanesulphonyloxy group can be synthesised starting from compounds **I** where R₁ is a hydroxy group by known procedures that include the use of trifluoromethanesulphonic anhydride or *N*-phenyltrifluoromethane-sulphonimide in aprotic solvents such as 1,2-dichloroethane or other chlorinated solvents or toluene, at a temperature in the range between 20°C and the temperature of reflux of the solvent (Hendickson J.B. et al., *Tetrahedron Letters,* 4607-4510 (1973)). The *N*-oxides of the compounds **I** may be synthesised by simple oxidation procedures known to those skilled in the art. The oxidation procedure described in P. Brougham in *Synthesis*, 1015-1017 (1987) allows differentiation of the two nitrogen atoms of the piperazine ring and both the N-oxides and N,N'-dioxides to be obtained.

Preparation of the phenylpiperazines **8** not yet known in the literature is well documented in the experimental part and uses synthetic procedures well known to those skilled in the art, which comprise the synthesis of the proper aniline through standard reactions and the subsequent cyclization with *bis*-(2-chloroethyl)amine to afford the piperazine following the method of Prelog (*Collect. Czech. Chem. Comm*. 5, 497-502 (1933)) or its variations (Elworthy T. R., *J. Med. Chem*. 40, 2674-2687 (1997).

### DETAILED SYNTHESIS OF THE COMPOUNDS OF THE INVENTION

### Example 1

### N-{3-[4-(5-Chloro-2-methoxyphenyl)-1-piperazinyl]-propyl}-7-oxo-5-phenyl-7H-thieno[3,2-b]pyran-3-carboxamide

### a) 1-(5-Chloro-2-methoxyphenyl)-4-[3-(N-phthalimido)-propyl]-piperazine (Compound 1A)

A mixture of 28.64 g of 1-(5-chloro-2-methoxyphenyl)-piperazine, 44.6 g of anhydrous potassium carbonate and *33.65* g of N-(3-bromopropyl)-phthalimide in 250 ml of acetonitrile was stirred at reflux for 8 hours. After cooling to 20-25°C, 800 ml of water was added under stirring and the resulting suspension was filtered by suction yielding a yellowish solid, which was washed with 300 ml of water and crystallised from methanol affording 46.5 g of the title compound, melting at 131-133°C.

¹H-NMR (200MHz, CDCl₃, δ): 7.78-7.82, m, 2H, phthalimide H3 and H6; 7.64-7.78, m, 2H, phthalimide H4 and H5; 6.92, dd, 1H, methoxyphenyl H4; 6.65-6.78, m, 2H, methoxyphenyl H3 and H6; 3.81, s, 3H, CH₃O; 3.71-3.89, m, 2H, CH₂N(CO)₂; 2.78-3.00, m, 4H, 3 and 5 piperazine CH₂s; 2.40-2.65, m, 6H, 2 and 6 piperazine CH₂s, CH₂CH₂CH₂N(CO)₂; 1.80-2.03, m, 2H, CH₂CH₂CH₂.

### b) 1-(3-Aminopropyl)-4-(5-chloro-2-methoxyphenyl)-piperazine trihydrochloride 2.15 H₂O (Compound 1B)

A solution of 20.7 g of Compound 1A and 8.6 ml of 85% hydrazine hydrate were stirred at reflux for 3.5 hours in 300 ml of ethanol. Afterwards, the reaction mixture was cooled to 20-25°C, diluted with 400 ml of water, acidified with 37% hydrochloric acid (pH = 1) and stirred for 30 minutes. The precipitated solid was collected by filtration and washed with 1N hydrochloric acid and then by water. The filtrate was concentrated by evaporation *in vacuo,* filtered, made basic by addition of 35% sodium hydroxide at 0-5°C and extracted with diethyl ether. The organic layer was washed with brine, dried on sodium sulphate and evaporated to dryness *in vacuo* affording 13.6 g (96%) of the title compound as a base. Acidification of the solution of the base in chloroform with more than three equivalents of 3N ethanolic hydrogen chloride followed by evaporation to dryness in vacuo and crystallisation of the residue from ethanol:diethyl ether 10:3, yielded the title compound, melting at 200-202°C.

¹H-NMR (200MHz, DMSO-d_{6,} δ): 11.20-11.50, br, 1H, NH⁺; 8.10-8.40, br, 3H, NH₃⁺; 6.85-7.10, m, 3H, phenyl H3, H4 and H6; 5.10, br, 5.3H, NH⁺, 2.15H₂O; 3.79, s, 3H, CH₃O; 3.35-3.65, m, 4H, 2 piperazine CH₂s; 3.03-3.35, m, 6H, 2 piperazine CH₂s, CH₂CH₂CH₂NH₃⁺; 2.80-3.03, m, 2H, CH₂CH₂CH₂NH₃⁺; 1.95-2.22, m, 2H, CH₂CH₂CH₂NH₃⁺.

### c) Methyl 2-acetyl-3-benzoyloxythiophene-4-carboxylate (Compound 1C)

3.48 ml of benzoyl chloride was added dropwise at 20-25°C to a solution of 5.0 g of methyl 2-acetyl-3-hydroxythiophene-4-carboxylate (prepared as described in *J. Chem. Soc. Perkin Trans I*, **1986,** 507) and 3.66 g of 4-dimethylaminopyridine in 100 ml of dichloromethane. The mixture was stirred for 2 hours; afterwards it was washed with 0.5N hydrochloric acid, water (2 x 20 ml), 2.5% aqueous sodium bicarbonate (2 x 40 ml) and water (2 x 20 ml). The organic layer was dried (sodium sulphate), evaporated to dryness *in vacuo* and purified by flash chromatography (chloroform:ethyl-acetate 100:1) affording 7.08 g of Compound 1C as a yellow deliquescent solid used in the next step without further purification.

¹H-NMR (200MHz, CDCl₃, δ): 8.36, s, 1H, thiophene H5; 8.20-8.42, m, 2H, phenyl H2, H6; 7.52-7.78, m, 3H, phenyl H3, H4, H5; 3.73, s, 3H, CH₃O; 2.50, s, 3H, CH₃CO.

### d) Methyl 2-(2-bromoacetyl)-3-benzoyloxythiophene-4-carboxylate (Compound 1D)

A solution of 1.28 ml of bromine in 24 ml of tetrachloromethane was added dropwise over a period of 10 minutes to a solution of 7.23 g of Compound 1C in 72 ml of tetrachloromethane stirred at reflux. After a further 5 minutes at reflux, the mixture was cooled to 20-25°C. The precipitated solid was filtered off and washed with cold tetrachloromethane affording 7 g (77%) of Compound 1D, melting at 115-118°C. The compound was contaminated with impurities 1C and methyl 2-(2,2-dibromoacetyl)-3-benzoyloxythiophene-4-carboxylate (2% and 6% mol. respectively, determined by ¹H-NMR spectroscopy), but could be used without further purification in the next reaction step.

¹H-NMR (200MHz, CDCl₃, δ): 8.43, s, 1H, thiophene H5; 8.20-8.42, m, 2H, phenyl H2, H6; 7.52-7.80, m, 3H, phenyl H3, H4, H5; 6.70, s, 0.06H, CHBr₂; 4.30, s, 1.84H, CH₂Br; 3.73, s, 3H, CH₃O; 2.50, s, 0.06H, CH₃CO.

### e) 2-[(3-Benzoyloxy-4-methoxycarbonyl)-2-thienyl]-2-oxoethyltriphenylphosphonium bromide hemihydrate (Compound 1E)

A solution of 6.9 g of compound 1D and 5.19 g of triphenylphosphine in 45 ml of acetonitrile was stirred at reflux for 4 hours and then cooled to 20-25°C. The precipitate was filtered off, affording 10.27 g (88%) of Compound 1E, melting at 150-152°C and pure enough to be used in further reactions. 0.27 g of this crude was crystallised from isopropanol affording 0.24 g of the analytical sample. M.p. (124)128-132°C.

¹H-NMR (200MHz, CDCl_{3,} δ): 8.38-8.50, m, 3H, PhCO H2, H6 and thienyl H5; 7.41-7.87, m, 18H, (C₆H₅)₃P and PhCO H3, H4, H5; 6.35, d, 2H, CH₂P; 3.71, s, 3H, CH₃O.

### f) Methyl 7-oxo-5-phenyl-7H-thieno[3,2-b]pyran-3-carboxylate (Compound 1F)

150 ml of 1M aqueous sodium carbonate was added to a solution of 10.07 g of Compound 1E in 200 ml of 1,2-dichloroethane and the mixture was stirred at 85°C for 11 hours. After cooling, the organic layer was separated, washed with water to neutrality, dried over anhydrous sodium sulphate and evaporated to dryness *in vacuo* affording 8.67 g of a crude residue. The crude was purified by flash chromatography (petroleum ether/ethyl acetate 6:4) yielding 4.1 g (92%) of Compound 1F, melting at 169-171°C. This was crystallised from methanol to give the analytical sample. M.p. 169-171°C.

¹H-NMR (200MHz, CDCl₃, δ): 8.50, s, 1H, H2; 7.95-8.05, m, 2H, phenyl H2, H6; 7.50-7.60, m, 3H, phenyl H3, H4, H5; 6.88, s, 1H, H6; 4.00, s, 3H, CH₃O.

### g) 7-Oxo-5-phenyl-7H-thieno[3,2-b]pyran-3-carboxylic acid (Compound 1G)

26 ml of 0.6N sodium hydroxide was added to a stirred solution of 3.82 g of Compound 1F in 174 ml of methanol and 87 ml of dioxane at 50°C. The mixture was then stirred at the same temperature for 20 minutes, cooled to 20-25°C, diluted with 280 ml of water, filtered and acidified with 1N hydrochloric acid to pH = 1. The suspension of the precipitate gel was stirred at 60°C for 2 hours, until a filtrable solid was obtained. This solid was filtered and dried to afford 3.4 g of the title compound, used in the next reaction step without further purification. It was crystallised from ethanol to yield the analytical sample, melting at 282-283°C.

¹H-NMR (200MHz, CDCl₃, δ): 13.39, bs, 1H, COOH; 8.50, s, 1H, H2; 8.00-8.05, m, 2H, phenyl H2, H6; 7.52-7.60, m, 3H, phenyl H3, H4, H5; 7.13, s, 1H, H6.

### h) N-{3-[4-(5-Chloro-2-methoxyphenyl)-1-piperazinyl]-propyl}-7-oxo-5-phenyl-7H-thieno[3,2-b]pyran-3-carboxamide

0.54 ml of 93% diethyl cyanophosphonate and 0.46 ml of triethylamine were added to a stirred solution of 0.82 g of Compound I G and 0.94 g of Compound 1B base in 15 ml of anhydrous dimethylformamide at 0°C. After 22 hours stirring at 20-25°C, the reaction mixture was poured into 150 ml of water. The mother liquors were decanted and the precipitated pasty solid was dissolved in 60 ml of chloroform, washed with water, dried over sodium sulphate and evaporated to dryness *in vacuo*. The crude was purified by flash chromatography (ethyl acetate/methanol 9:1). Evaporation afforded the pure title compound (1.2 g; 74%), which was crystallised from ethyl acetate. M.p. 165-166.5°C.

¹H-NMR (200MHz, CDCl_{3,} δ): 8.45, s, 1H, H2; 7.90-8.02, m, 2H, phenyl H2, H6; 7.55-7.62, m, 3H, phenyl H3, H4, H5; 7.45, t, 1H, CONH; 6.95, dd, 1H, chlorophenyl H4; 6.83, s, 1H, H6; 6.65-6.75, m, 2H, chlorophenyl H3, H6; 3.81, s, 3H, CH₃O; 3.66, dt, 2H, CONHCH₂; 2.74-2.92, m, 4H, 2 piperazine CH₂s; 2.48-2.54, m, 6H, CH₂N and 2 piperazine CH₂s; 1.80-2.00, m, CH₂CH₂CH₂.

### Example 2

### N-{3-[4-(2-Methoxyphenyl)-1-piperazinyl]-propyl}-7-oxo-5-phenyl-7H-thieno[3,2-b]pyran-3-carboxamide

The title compound was prepared as described in Example 1h, but substituting 1-(3-aminopropyl)4-(2-methoxyphenyl)-piperazine (prepared as described in GB 2161807) for Compound 1B. After pouring the reaction mixture into water and extracting with ethyl acetate, the combined organic layers were washed with water (3 x 80 ml), dried (sodium sulphate) and evaporated to dryness *in vacuo.* The crude was purified by flash chromatography (ethyl acetate/methanol 8.5:1.5). The residue obtained by evaporation of the collected fractions containing the pure title compound (1.4 g; 77 %) was crystallised from ethyl acetate affording the title compound melting at 161-162°C.

¹H-NMR (200MHz, CDCl₃, δ): 8.41, s, 1H, H2; 7.90-8.02, m, 2H, phenyl H2, H6; 7.50-7.65, m, 4H, NHCO and phenyl H3, H4, H5; 6.80, s, 1H, H6; 6.70-7.05, m, 4H, CHs of methoxyphenyl ring; 3.83, s, 3H, CH₃O; 3.66, dt, 2H, CONHCH₂; 2.80-3.00, m, 4H, 2 piperazine CH₂s; 2.48-2.62, m, 6H, CH₂N and 2 piperazine CH₂s; 1.80-2.00, m, 2H, CH₂CH₂CH₂.

### Example 3

### 5-Cyclohexyl-N-[3-[4-(2-methoxyphenyl)-1-piperazinyl]propyl}-7-oxo-7H-thieno[3,2-b]pyran-3-carboxamide

### a) Methyl 2-acetyl-3-cyclohexanecarbonyloxythiophene-4-carboxylate (Compound 3A)

This compound was prepared as described for compound 1C of Example 1, but using cyclohexanecarbonyl chloride instead of benzoyl chloride. The crude was purified by flash chromatography (petroleum ether:ethyl acetate gradient from 9:1 to 7:3) to afford Compound 3A (80%).

¹H-NMR (200MHz, CDCl₃, δ): 8.30, s, 1H, thiophene H5; 3.80, s, 3H, CH₃O; 2.50, s, 3H, CH₃CO; 1.00-3.00, m, 11H, cyclohexane CHs.

### b) Methyl 2-(2-bromoacetyl)-3-cyclohexanecarbonyloxythiophene-4-carboxylate (Compound 3B)

A solution of 0.70 ml of bromine in 3.45 ml of acetic acid was added dropwise over a period of 60 minutes to a solution of 3.56 g of Compound 3A in 34.5 ml of acetic acid stirred at 20-25°C. After stirring for a further 2.5 hours at 20-25°C, the mixture was poured into iced water and extracted with diethyl ether (2 x 80 ml). The combined organic layers were washed with water (2 x 80 ml), 10% aqueous sodium carbonate (100 ml) and water (3 x 80 ml), dried over sodium sulphate and evaporated to dryness *in vacuo.* The crude was purified by flash chromatography (n-hexane:chloroform 6:4) to yield 1.31 g (29 %) of Compound 3B.

¹H-NMR (200MHz, CDCl₃, δ): 8.36, s, 1H, thiophene H5; 4.29, s, 2H, CH₂Br; 3.83, s, 3H, CH₃O; 2.65-2.80, m, 1H, cyclohexane CH; 2.15-2.25, m, 2H, 2, 6 cyclohexane CHs (eq.); 1.85-1.95, m, 2H, 2, 6 cyclohexane CHs (ax.); 1.25-1.80, m, 6H, 3, 4, 5 cyclohexane CH₂s.

### C) 2-[(3-Cyclohexanecarbonyloxy-4-methoxycarbonyl)-2-thienyl]-2-oxoethyltriphenylphosphonium bromide (Compound 3C)

A solution of 0.20 g of compound 3B and 0.13 g of triphenylphosphine in 1.25 ml of acetonitrile was stirred at reflux for 2.5 hours and then cooled to 0-5°C. The precipitate was filtered off, washing on the filter with a 2:1 mixture of ethyl acetate:acetonitrile followed by ethyl acetate, affording 0.19 g (59 %) of Compound 3C melting at 165-167°C.

¹H-NMR (200MHz, CDCl₃, δ): 8.31, s, 1H, thiophene H5; 7.55-8.00, m, 15H, (C₆H₅)₃P; 6.35, d, 2H, CH₂P; 3.79, s, 3H, CH₃O; 2.60-2.75, m, 1H, cyclohexane CH; 1.95-2.05, m, 2H, 2,6 cyclohexane CHs (eq.); 1.10-1.70, m, 8H, other cyclohexane CHs.

### d) Methyl 5-cyclohexyl-7-oxo-7H-thieno[3,2-b]pyran-3-carboxylate (Compound 3D)

A mixture of 0.16 g of Compound 3C, 2 ml of 1,2-dichloroethane and 2 ml of 1M aqueous sodium carbonate was heated at 45°C for 36 hours. After cooling to 20-25°C, 5 ml of chloroform was added, the organic layer was washed with water (2 x 10 ml), dried on anhydrous sodium sulphate and evaporated to dryness *in vacuo*. The crude was purified by flash chromatography (petroleum ether:ethyl acetate 1:1) yielding 0.05 g (68%) of Compound 3D as a white solid, melting at 114-119°C.

¹H-NMR (200MHz, CDCl₃, δ): 8.43, s, 1H, H2; 6.20, s, 1H, H6; 3.94, s, 3H, COOCH₃; 2.55-2.70, m, 1H, cycloexane CH; 1.15-2.15, m, 10H, cycloexane CH₂s.

### e) 5-Cyclohexyl-7-oxo-7H-thieno[3,2-b]pyran-3-carboxylic acid (Compound 3E)

0.3 ml of 1N sodium hydroxide and 1.0 ml of water were added to a stirred solution of 0.040 g of Compound 3D in 1.8 ml of methanol and 0.9 ml of 1,4-dioxane at 20-25°C. The mixture was heated at 50°C for 3.5 hours. After cooling to 20-25°C, the mixture was diluted with water and acidified to pH 1 with 3N hydrochloric acid. The precipitated solid was collected by filtration and washed with water to afford 0.028 g (73.5%) of the title compound, melting at 269-275°C.

¹H-NMR (200MHz, DMSO-d₆, δ): 13.30, bs, 1H, COOH; 8.78, s, 1H, H2; 6.23, s, 1H, H6; 2.55-2.70, m, 1H, cycloexane CH; 1.10-2.05, m, 10H, cycloexane CH₂s.

### f) 5-Cyclohexyl-N-{3-[4-(2-methoxyphenyl)-1-piperazinyl]-propyl}-7-oxo-7H-thieno[3,2-b]pyran-3-carboxamide

The title compound was prepared as described in Example 2, but substituting Compound 3E for Compound 1G. The crude was purified by flash chromatography (ethyl acetate:2.7N methanolic ammonia 95:5). The residue, obtained after solvent evaporation from the collected fractions containing the pure title compound (0.03 g; 73%) was dissolved in 5 ml of methanol and the opalescent solution clarified with charcoal. Solvent evaporation afforded the pure title compound as a yellow pasty solid (67%).

¹H-NMR (200MHz, CDCl₃, δ): 8.41, s, 1H, H2; 7.15, t, 1H, NH; 6.85-7.10, m, 4H, methoxyphenyl CHs; 6.21, s, 1H, H6; 3.86, s, 3H, OCH₃; 3.60, q, 2H, NHCH₂; 3.00-3.15, m, 4H, 2 piperazine CH₂s; 2.55-2.80, m, 7H, 2 piperazine CH₂s, cyclohexane CH and CH₂CH₂CH₂N; 2.05, dt, 2H, CH₂CH₂CH₂; 1.20-1.95, m, 10H, cyclohexane CH₂s.

### Example 4

### N-{3-[4-(2-Methoxyphenyl)-1-piperazinyl]-propyl}-7-oxo-5-trifluoromethyl-7H-thieno[3,2-b]pyran-3-carboxamide

### a) Methyl 7-oxo-5-trifluoromethyl-7H-thieno[3,2-b]pyran-3-carboxylate (Compound 4A)

3.95 ml of trifluoroacetic anhydride and 9.2 ml of 1,8-diazabicyclo[5.4.0]undec-7-ene (DBU) were added to a mixture of 4.10 g of methyl 2-acetyl-3-hydroxythiophene-4-carboxylate and 14 ml of pyridine at 0-5°C. The mixture was heated at 80°C for 27 hours. During this period three further additions of trifluoroacetic anhydride (9.9 ml total) and DBU (9.2 ml) were made. After cooling to 20-25°C the mixture was poured into a mixture of ice (250 g) and 37% hydrochloric acid (50 ml) and extracted with ethyl acetate (2 x 80 ml). The combined organic layers were washed with water, dried over sodium sulphate and evaporated to dryness *in vacuo*. The residue was taken up with petroleum ether:ethyl acetate 7:3 and filtered. The filtrate was purified by flash chromatography (petroleum ether:ethyl acetate gradient from 7:3 to 0:1). The residue was dissolved in diethyl ether, washed with 5% aqueous sodium carbonate and water, dried over sodium sulphate and evaporated to dryness *in vacuo* to afford the title product (22%) melting at 148-158°C, which could be used in the next step without further purification. The analytical sample was obtained by crystallisation from ethanol. M.p. 163-164°C

¹H-NMR (200MHz, CDCl₃, δ): 8.58, s, 1H, H2; 6.80, s, 1H, H6; 3.96, s, 3H, COOCH₃.

### b) 7-Oxo-5-trifluoromethyl-7H-thieno[3,2-b]pyran-3-carboxylic acid (Compound 4B)

A mixture of 0.70 g of Compound 4A, 5.6 ml of dioxane and 8.4 ml of 9N hydrochloric acid was stirred at reflux for 75 minutes. After cooling to 20-25°C, the precipitated solid was filtered off, washed with dioxane:water 1:1.5 and water to afford 0.46 g of the title compound as a grey solid melting at 249-251°C.

¹H-NMR (200MHz, DMSO-d₆, δ): 13.50, bs, 1H, COOH; 8.25, s, 1H, H2; 7.19, s, 1H, H6.

### c) N-{3-[4-(2-Methoxyphenyl)-1-piperazinyl]-propyl}-7-oxo-5-trifluoromethyl-7H-thieno[3,2-b]pyran-3-carboxamide

The title compound was prepared as described in Example 2 but substituting Compound 4B for Compound 1G. The crude was purified by flash chromatography (ethyl acetate:2.7N ammonia in methanol 95:5) affording the title compound as a light-brown solid melting at 170-177°C (33%).

¹H-NMR (200MHz, CDCl₃, δ): 8.55, s, 1H, H2; 7.10, t, 1H, NH; 6.85-7.10, m, 4H, methoxyphenyl CHs; 6.80, s, 1H, H6; 3.88, s, 3H, OCH₃; 3.60, q, 2H, NHCH₂; 2.90-3.15, m, 4H, 2 piperazine CH₂s; 2.45-2.80, m, 6H, 2 piperazine CH₂s, CH₂CH₂CH₂N; 1.88, dt, 2H, CH₂CH₂CH₂.

### Example 5

### 7-Oxo-5-phenyl-N-{3-[4-[2-(2,2,2-trifluoroethoxy)-phenyl]-1-piperazinyl]-propyl}-7H-thieno[3,2-b]pyran-3-carboxamide

### a) N-(3-Chloropropyl)-7-oxo-5-phenyl-7H-thieno[3,2-b]pyran-3-carboxamide (Compound 5A)

This compound was prepared as described in Example 1, but with the substitution of 3-chloropropylamine hydrochloride for Compound 1B and doubling the amount of triethylamine used. After dilution with water, the precipitated solid was filtered and washed with cold-water:dimethylformamide 2:1 and then with water on the filter. The solid was then suspended in 10% aqueous sodium carbonate, stirred, filtered and washed with water to neutrality. Drying at 70°C *in vacuo* afforded the title compound (95%).

¹H-NMR (200MHz, CDCl₃, δ): 8.52, s, 1H, H2; 7.75-7.85, m, 2H, phenyl H2, H6; 7.50-7.60, m, 3H, phenyl H3, H4, H5; 7.00, s, 1H, NH; 6.80, s, 1H, H6; 3.65-3.80, m, 4H, CH₂CH₂CH₂; 2.15, dt, 2H, CH₂CH₂CH₂.

### b) 7-Oxo-5-phenyl-N-[3-[4-[2-(2,2,2-trifluoroethoxy)phenyl]-1-piperazinyl]propyl]-7H-thieno[3,2-b]pyran-3-carboxamide

A mixture of 0.17 g of Compound 5A, 0.13 g of 1-[2-(2,2,2-trifluoroethoxy)-phenyl]-piperazine (prepared as described in EP 0748800) and 0.07 g of potassium carbonate was heated at 200°C for 20 minutes. After cooling to 20-25°C, the crude residue was purified by flash chromatography (ethyl acetate:methanol gradient from 95:5 to 9:1) to afford 0.193 g (70%) of the title compound. M.p. 152-158°C.

¹H-NMR (200MHz, CDCl₃, δ): 8.45, s, 1H, H2; 7.80-7.95, m, 2H, phenyl H2, H6; 7.50-7.65, m, 4H, CONH, phenyl H3, H4, H5; 6.80, s, 1H, H6; 6.75-7.10, m, 4H, trifluoroethoxyphenyl CHs; 4.44, q, 2H, CH₂O; 3.66, dt, 2H, CONHCH₂; 2.90-3.05, m, 4H, 2 piperazine CH₂s; 2.50-2.70, m, 6H, CH₂N and 2 piperazine CH₂s; 1.80-2.00, m, 2H, CH₂CH₂CH₂.

### Example 6

### N-{3-[4-[2-Methoxy-5-(2,2,2-trifluoroethoxy)phenyl]-1-piperazinyl]-propyl}-7-oxo-5-phenyl-7H-thieno[3,2-b]pyran-3-carboxamide

### a) 1-t-Butoxycarbonyl-4-(5-hydroxy-2-methoxyphenyl)-piperazine (Compound 6A)

A solution of 8 g of 1-(5-hydroxy-2-methoxyphenyl)-piperazine dihydrobromide and 3.17 g of anhydrous potassium carbonate in 30 ml of water was evaporated to dryness *in vacuo*. 100 ml of anhydrous tetrahydrofuran and 5.18 g of 97% di-t-butyl dicarbonate were added to the residue and the mixture was stirred at 20-25°C for 2 hours, followed by addition of 100 ml of anhydrous tetrahydrofuran. The suspension was filtered and the solvent removed from the filtrate *in vacuo*. The residue was dissolved in 200 ml of chloroform. The solution was washed with 3 x 50 ml of 5% sodium bicarbonate and with 2 x 50 ml of water, and dried over sodium sulphate. The solvent was removed at reduced pressure and the residue was purified by flash chromatography (petroleum ether:ethyl acetate 75:25) to give 1.91 g (28.7%) of Compound 6A and 1.58 g (35.7%) of 1-*t*-butoxycarbonyl-4-(5-*t*-butoxycarbonyloxy-2-methoxyphenyl)-piperazine. A solution of this by-product in 40 ml of methanol and 6 ml of 1N sodium hydroxide was maintained overnight at 20-25°C. The mixture was neutralised with acetic acid; the solvent was removed at reduced pressure and the residue was dissolved in 40 ml of chloroform. After washing with 3 x 10 ml of water, the organic layer was dried over sodium sulphate and the solvent evaporated *in vacuo* to recover an additional 1.15 g (17.2%) of Compound 6A as a thick oil (total yield 45.9%).

¹H-NMR (200MHz, CDCl₃, δ): 6.70, d, 1H, H3 of phenyl ring; 6.45-6.53, m, 2H, H4 and H6 of phenyl ring; 5.77, s, 1H, OH; 3.78, s, 3H, CH₃O; 3.48-3.68, m, 4H, 2 piperazine CH₂s; 2.82-3.05, m, 4H, 2 piperazine CH₂s; 1.48, s, 9H, (CH₃)₃C.

### b) 1-t-Butoxycarbonyl-4-[2-methoxy-5-(2,2,2-trifluoroethoxy)phenyl]-piperazine (Compound 6B)

A stirred mixture of 2.83 g of Compound 6A, 6.05 g of cesium carbonate and 2.95 g of 2,2,2-trifluoroethyl p-toluenesulphonate in 60 ml of acetonitrile was refluxed for 16 hours. The solvent was evaporated off at reduced pressure; 90 ml of brine was added to the residue and the mixture was extracted with 3 x 40 ml of ethyl acetate. The organic layer was washed with 3 x 20 ml of water and 20 ml of brine and dried over sodium sulphate. The solvent was removed at reduced pressure and the residue purified by flash chromatography (petroleum ether:ethyl acetate gradient 95:5 to 80:20). The solvents were removed *in vacuo* to give 1.86 g (52%) of Compound 6B as a white solid. M.p. (98) 102-105°C.

¹H-NMR (200MHz, CDCl₃, δ): 6.77, d, 1H, H3 of phenyl ring; 6.45-6.63, m, 2H, H4 and H6 of phenyl ring; 4.28, q, 2H, CF₃CH₂O; 3.84, s, 3H, CH₃O; 3.53-3.68, m, 4H, 2 piperazine CH₂s; 2.90-3.06, m, 4H, 2 piperazine CH₂s; 1.48, s, 9H, (CH₃)₃C.

### c) 1-[2-Methoxy-5-(2,2,2-trifluoroethoxy)-phenyl]-piperazine · 1.9 hydrochloride (Compound 6C)

A solution of 2.42 ml of trifluoroacetic acid in 30 ml of anhydrous dichloromethane was added dropwise at 3-5°C to a stirred solution of 1.17 g of Compound 6B in 40 ml of anhydrous dichloromethane. The mixture was maintained overnight at 20-25°C, washed with 2 x 30 ml of 2N sodium hydroxide and extracted with 3 x 15 ml of 2N hydrochloric acid. The aqueous acid layer was washed with 2 x 20 ml of diethyl ether, alkalinised with 37% sodium hydroxide at 5-10°C and extracted with 3 x 30 ml of diethyl ether. The organic layer was dried over sodium sulphate and the solvent was removed *in vacuo* to give 0.78 g (89%) of compound 6C base as a thick oil. A solution of the base in diethyl ether was treated with coal, filtered and acidified by addition of 3.6N HCI in diethyl ether to give the hydrochloride salt, which was recovered by filtration and crystallised from acetonitrile and ethanol to yield the analytical sample. M.p. (188) 202-208°C (dec.).

¹H-NMR (200MHz, DMSO-d₆, δ): 9.18, bs, 2.9H, NH₂⁺ and NH⁺; 6.90, d, 1H, phenyl H3; 6.67, dd, 1H, phenyl H4; 6.59, d, 1H, phenyl H6; 4.66, q, 2H, CF₃CH₂O; 3.74, s, 3H, CH₃O; 3.18, bs, 8H, piperazine CH₂s.

### d) N-{3-[4-[2-Methoxy-5-(2,2,2-trifluoroethoxy)-phenyl]-1-piperazinyl]-propyl}-7-oxo-5-phenyl-7H-thieno[3,2-b]pyran-3-carboxamide

This compound was prepared as described above, in Example 5b, with the exception that Compound 6C was used in place of 1-[2-(2,2,2-trifluoroethoxy)-phenyl]-piperazine. After cooling to 20-25°C, the crude residue was purified by flash chromatography (ethyl acetate:2N ammonia in methanol 98:2) to afford the title compound (60%). M.p. 156-158°C.

¹H-NMR (200MHz, CDCl₃, δ): 8.50, s, 1H, H2; 7.80-7.95, m, 2H, phenyl H2, H6; 7.40-7.80, m, 4H, CONH, phenyl H3, H4, H5; 6.85, s, 1H, H6; 6.75, d, 1H, trifluoroethoxyphenyl H3; 6.40-6.55, m, 2H, trifluoroethoxyphenyl H4, H6; 4.30, q, 2H, CH₂O; 3.80, s, 3H, CH₃O; 3.65, dt, 2H, CONHCH₂; 2.50-3.10, m, 10H, piperazine CH₂s and CH₂N; 1.85-2.10, m, 2H, CH₂CH₂CH₂.

### Example 7

### N-{3-[4-[4-Fluoro-2-(2,2,2-trifluoroethoxy)-phenyl]-1-piperazinyl]-propyl}-7-oxo-5-phenyl-7H-thieno[3,2-b]pyran-3-carboxamide

This compound was prepared as described in Example 5b, except that 1-[4-fluoro-2-(2,2,2-trifluoroethoxy)-phenyl]-piperazine (prepared as described in EP 0748800) was used in place of 1-[2-(2,2,2-trifluoroethoxy)-phenyl]-piperazine. After cooling to 20-25°C, the crude residue was purified by flash chromatography (ethyl acetate:2N ammonia in methanol 95:5) to afford the title compound (74%). M.p. 189-191°C.

¹H-NMR (200MHz, CDCl₃, δ): 8.45, s, 1H, H2; 7.80-7.95, m, 2H, Phenyl H2, H6; 7.45-7.65, m, 4H, CONH, phenyl H3, H4, H5; 6.80, s, 1H, H6; 6.65-6.75, m, 2H, trifluoroethoxyphenyl CHs; 6.60, dd, 1H, trifluoroethoxyphenyl CH; 4.35, q, 2H, CH₂O; 3.65, dt, 2H, CONHCH₂; 2.80-3.00, m, 4H, 2 piperazine CH₂s; 2.50-2.70, m, 6H, CH₂N and 2 piperazine CH₂s; 1.90-2.00, m, 2H, CH₂CH₂CH₂.

### Example 8

### Determination of binding affinity for cloned α₁ adrenoceptors and 5-HT_{1A} serotoninergic receptors

Determination of affinity for cloned human α₁-adrenoceptor subtypes was performed in membranes from CHO cells (Chinese hamster ovary cells) transfected by electroporation with DNA expressing the genes encoding each α₁-adrenoceptor subtype. Cloning and stable expression of the α₁-adrenoceptor genes were performed as previously described (Testa et al., *Pharmacol. Comm*. 6, 79-86 (1995)). The CHO-cell membranes were incubated in 50 nM Tris, pH 7.4, with 0.2 nM [³H]prazosin, in a final volume of 1.02 ml for 30 minutes at 25°C, in the absence or presence of competing drugs (1 pM-10 µM). Non-specific binding was determined in the presence of 10 µM phentolamine. Incubation was stopped by addition of ice-cold Tris buffer and rapid filtration through 0.2%-polyethyleneimine-pretreated Schleicher & Schuell GF52 filters.

Clone-G-21 genome for the human 5-HT_{1A} serotoninergic receptor was stably transfected in a human cell line (HeLa) (Fargin et al., *J. Biol. Chem*. 284, 14848-14852 (1989)). The HeLa cells were grown as monolayers in Dulbecco's modified Eagle medium (DMEM), supplemented with 10% foetal calf serum and gentamicin (100 g/ml), 5% CO₂ at 37°C. The cells were detached from the growth flask at 95% confluence by a cell scraper and were lysed in ice-cold Tris-5-mM and EDTA-5-mM buffer (pH 7.4). The homogenates were centrifuged at 40000 x g x 20 minutes and the membranes were resuspended in a small volume of ice-cold Tris-5-mM and EDTA-5-mM buffer (pH 7.4) and immediately frozen and stored at -70°C until use.

On the experiment day, the cell membranes were resuspended in a binding buffer of 50 mM Tris (pH 7.4), 2.5 mM MgCl₂, 10 µM pargyline (Fargin et al., Nature 335, 358-360 (1988)). The membranes were incubated in a final volume of 1 ml for 30 minutes at 30°C with 1.2 nM [³H]8-OH-DPAT, in the absence or presence of competing drugs; nonspecific binding was determined in the presence of 10 µM 5-HT. The incubation was stopped by addition of ice-cold Tris buffer and rapid filtration through 0.2%-polyethyleneimine-pretreated Schleicher & Schuell GF52 filters.

Inhibition of specific binding of the radioligands by the test drugs was analysed to estimate the IC₅₀ value by using the non-linear curve-fitting program Allfit (De Lean et al., *Am. J. Physiol.* 235, E97-E102 (1978)). The IC₅₀ value was converted to an affinity constant (Ki) by the equation of Cheng & Prusoff (Biochem. Pharmacol. 22, 3099-3108 (1973)). Data were expressed as mean of Ki.

The compounds of the invention exhibited the desired potency and selectivity at α₁-adrenoceptor, as shown in Table 1.

**Table 1**

| *Affinity (Ki, nM) of the different compounds tested for recombinant α*_{*1*}*-adrenoceptor subtypes and the 5-HT1A receptor.* | | | | |
|---|---|---|---|---|
| **Example** | **Cloned receptors** | | | |
| | **α**_{**1a**} | **α**_{**1b**} | **α**_{**1d**} | **5-HT**_{**1A**} |
| 1 | 0.58 | 2.53 | 4.12 | |
| 2 | 0.10 | 7.52 | 2.46 | 4.48 |
| 4 | 0.60 | 23.16 | 3.60 | 26.21 |
| 5 | 0.045 | 4.34 | 1.01 | 7.59 |
| 6 | 3.19 | 39.31 | 48.17 | 1081.00 |
| 7 | 0.17 | 3.47 | 2.45 | 88.54 |
| Compound A | 0.60 | 3.29 | 2.84 | 4.53 |
| Prazosin | 0.61 | 0.42 | 0.23 | >10000 |

### Example 9

### Functional affinity for the α_{1L}-adrenoceptors

The functional α₁-antagonistic activity of the test compounds against noradrenaline- (NA-) induced contractions of rabbit aorta pretreated with chloroethylclonidine (α_{1L} receptor) was evaluated according to the method of Testa et al. (*J. Pharmacol. Exp. Ther.* 281, 1284-1293 (1997)). Adult male New Zealand rabbits were sacrificed by cervical dislocation. The aorta was removed, placed in Krebs-Henseleit buffer and dissected free of adhering tissue. Rings were prepared from each artery (8 rings per aorta, about 4-5 mm wide) and suspended in 20 ml organ bath containing Krebs bicarbonate buffer of the following composition (mM): NaCl 112.0, KCl 5.0, CaCl₂ 2.5, KH₂PO₄ 1.0, MgSO₄ 1.2, NaHCO₃ 12.0 and glucose 11.1, equilibrated at 37° C with 95% O₂: 5% CO₂. Desmethylimipramine (0.1 µm) and corticosterone (1 µM) to block neuronal and extraneuronal uptake of NA, (±)-propranol (1 µM) to block β adrenoceptors and yohimbine (0.1 µM) to block α₂ adrenoceptors, were added to the buffer. The tissues were subjected to a passive load of 2 g and the developed tension was determined using isometric transducers (Basile 7003).

The preparations were allowed to equilibrate for 60 minutes and then primed every 30 minutes with 10 µM NA for three times. The aortic rings were then incubated with the alkylating agent chloroethylclonidine (5 x 10⁻⁵ M) for 30 minutes and then washed extensively three times (in 0.5 hours) before constructing the NA-concentration/response curve. After washout of NA and re-equilibration of the tissue (45 minutes), the drug to be tested was added and, after 30 minutes, a second NA-cumulative-concentration/response curve constructed. Each antagonist concentration was tested using 2-3 aortic rings from different rabbits.

Dose ratios (i.e., the ratio between the concentrations of norepinephrine required to produce a half-maximal response in the presence and in the absence of the test antagonist) were calculated at each concentration of the compounds. The logarithm of these dose ratio -1 was plotted against the logarithm of the compound concentrations (Schild plot) to evaluate the affinity constant Kb. When only one or two concentrations of the test compounds were utilised, the apparent Kb value was calculated using the formula: Kb = [B]/(DOSE RATIO-1), where B is the antagonist concentration.

### RESULTS

The compounds tested showed good affinity for the α_{1L} adrenoceptor subtype. The data are expressed as pKb in Table 2.

**Table 2**

| *Functional affinity of the tested compounds for the α*_{*1L*} *adrenoceptor subtype.* | |
|---|---|
| **Example** | **pKb** |
| 1 | 8.17 |
| 2 | 8.85 |
| 4 | 7.92 |
| 5 | 9.12 |
| 7 | 8.66 |
| Compound A | 8.64 |
| Prazosin | 8.11 |

### Example 10

### Effects on urethral contractions induced by noradrenaline injection and blood pressure in dogs after i.v. administration

The experiments were performed according to the method of Imagawa et al. (J. Pharmacol. Methods 22, 103-111 (1989)), with substantial modifications, as follows: adult male beagle dogs, weighing 8-10 kg, were anaesthetised with pentobarbital sodium (30 mg/kg i.v. and 2 mg/kg/h i.v.), intubated and spontaneously ventilated with room air. In order to monitor systemic blood pressure (BP), a polyethylene (PE) catheter was introduced into the aortic arch through the left femoral artery. A collateral of the left femoral vein was cannulated for infusion of anaesthetic, and the right femoral vein was cannulated for administration of compounds. For intraarterial (i.a.) injection of noradrenaline (NA), a PE catheter was introduced into the lower portion of the abdominal aorta via the right external iliac artery. Through such procedure, NA was selectively distributed to the lower urinary tract. A paramedian vertical suprapubic incision extending from the base of the pelvis to the mid-abdominal region was made and the bladder and the prostate were exposed. The bladder was manually emptied with a syringe. Prostatic urethral pressure was monitored with a Mikro-tip catheter (5F) introduced into the bladder via the external urethral meatus, and withdrawn until the pressure transducer was positioned in the prostatic region of the urethra. A ligature was secured between the neck of the bladder and urethra to isolate the response of the latter and to avoid any interaction with the bladder. Another ligature was put around the Mikro-tip catheter at the external meatus, to secure the catheter itself.

After a stabilising period following the surgical procedure (30 minutes), in which arterial and prostatic urethral pressures were continuously monitored as basal values, i.a. administration of NA was made at intervals of 20 minutes.

The doses of NA chosen were such to produce an increase of at least 100% in urethral pressure. The test compounds were administered i.v. in a cumulative manner with intervals of 15-20 minutes between administrations. I.a. injections of NA were repeated 5 minutes after every dosing of test compound with intervals of about 10 minutes between stimulations. In order to compare the effects of the administered compound, dose/response curves (log dose transformation) were constructed by computing, at the peak effect, the percent decrease in diastolic blood pressure and percent inhibition of the increase in urethral pressure induced by NA. Linear regression equations were then used in order to evaluate the theoretical effectiveness as ED₂₅ (the effective dose inducing a 25% decrease in diastolic blood pressure) and ID₅₀ (the dose inhibiting by 50% the increase in urethral pressure).

### RESULTS

The effects obtained after iv. administration of the compounds of examples 1, 2 and 5 are shown in Table 3. The results concerning the effects obtained after injection of prazosin and Rec 15/2739 are also shown in Table 3.

**Table 3**

| *Data represent the active doses (expressed in* µ*g*/*kg) inhibiting 50% of the urethral contractions (UC) induced by noradrenaline (NA), the active doses (expressed in* µ*g*/*kg) in lowering diastolic blood pressure (DBP) and the ratio (DBP*/*UC) between the active doses.* | | | |
|---|---|---|---|
| **Compound** | **UC ID**_{**50**} **NA** | **DBP ED**_{**25**} | **Ratio** |
| 1 | 5.3 | 280 | 52.8 |
| 2 | 1.8 | 35.5 | 19.7 |
| 5 | 2.7 | >1000 | >370 |
| Prazosin* | 3.6 | 6.6 | 1.83 |
| Compound A* | 2.4 | 243 | 101.2 |

| | | | |
|---|---|---|---|
| * Data from Leonardi et al., *J Pharmacol Exp Ther* 281, 1272-1283 (1997). | | | |

The pharmacological results confirm that the compounds of the invention are α₁-adrenoceptor antagonists with good selectivity for the α₁ adrenoceptor, in particular compared to the 5-HT_{1A} receptor, and good affinity also for the α_{1L} subtype, as far as *in vitro* data are concerned.

The *in vivo* pharmacological results confirm the high uroselectivity of the compounds of the invention and justify their possible use in the treatment of obstructive diseases of the lower urinary tract, including BPH.

### Effective Amounts

The following represent guidelines to effective oral, parenteral or intravenous dose ranges, expressed in mg/kg of body weight per day, for use in obstructive disorders of the lower urinary tract:

| | |
|---|---|
| General | 0.001-20 |
| Preferred | 0.05-3 |
| Most preferred | 0.5-2 |

The most-preferred values refers to oral dosing. Intravenous dosages should be 10 to 100 fold lower. Selective-use dosages, i.e. dosages that are active in the lower urinary tract without a substantial effect on blood pressure, depend on the particular compound employed. Generally, in the case of a compound selective in inhibiting urethral contraction, up to four times the amount of the ED₅₀ used in inhibiting urethral contraction can be administered without substantial effect on blood pressure. Further refinements and optimisation of dosages are possible using no more than routine experiments. The active compounds of the invention may be orally administered, for example, with an inert diluent or with an edible carrier, or they may be enclosed in gelatine capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but the amount of active ingredient may be varied depending upon the particular form and may conveniently be between 5% and about 70% of the weight of the unit. The amount of active compound in such compositions is such that a suitable dosage will be obtained although the desired dosage can be obtained by administering a plurality of dosage forms. The preferred compositions and preparations according to the invention are prepared so that an oral dosage unit form contains between 1.0-300 milligrams of active compound. The tablets, pills, capsules, troches and the like may also contain, for example, the following ingredients: a binder such as microcrystalline cellulose, gum tragacanth or gelatine; an excipient such as starch or lactose; a disintegrating agent such as alginic acid, sodium starch glycolate, cornstarch and the like; a lubricant such as magnesium stearate or hydrogenated castor oil, a glidant such as colloidal silicon dioxide; and a sweetening agent such as sucrose or saccharin may be added, or a flavouring agent such as peppermint, methyl salicylate, or orange flavouring. When the dosage unit form is a capsule, it may contain, in addition to materials of the above type, a liquid carrier such as a fatty oil. Other dosage unit forms may contain other various materials which modify the physical form of the dosage unit, for example coatings. Thus, tablets or pills may be coated with sugar, shellac, or other enteric-coating agents. A syrup may contain, in addition to the active compounds, sucrose as a sweetening agent and certain preservatives, dyes, colouring and flavours. The materials used in preparing these various compositions should be pharmaceutically pure and nontoxic in the amounts used. For the purpose of parenteral therapeutic administration, the active compounds of the invention may be incorporated into a solution or suspension. These preparations should contain at least 0.1% of active compound, but it may be varied between 0.5 and about 30% of the weight thereof. The amount of active compound in such compositions is such that a suitable dosage will be obtained. The preferred compositions and preparations according to the present inventions are prepared so that a parenteral dosage unit contains between 0.2 to 100 milligrams of active compound. Solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulphite; chelating agents such as ethylenediaminetetraacetic acid; buffers such as acetates; citrates or phosphates and agents for the adjustment of tonicity such as sodium chloride or dextrose. The parenteral multiple-dose vials may be of glass or plastics material. Additional compositions suitable for administration by various routes and containing compounds according to the present invention are also within the scope of the invention.

Dosage forms, additional ingredients and routes of administration contemplated herein include those disclosed in US 4089969 and US 5091182.

## Claims

1. A compound having the general formula I 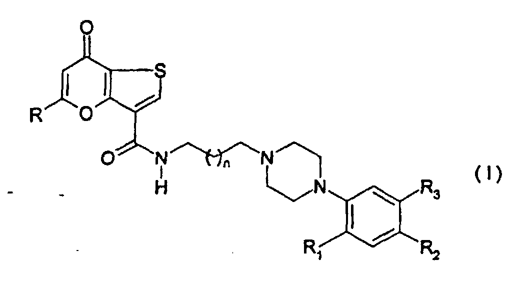 wherein
R represents an aryl, cycloalkyl or polyhaloalkyl group,
R₁ represents an alkyl, alkoxy, polyfluoroalkoxy, hydroxy or trifluoromethanesulphonyloxy group,
each of R₂ and R₃ independently represents a hydrogen or halogen atom or an alkoxy or polyfluoroalkoxy group, and
n is 0, 1 or 2,
or an N-oxide or a pharmaceutically acceptable salt of such a compound.

2. A compound according to claim 1, wherein R represents a phenyl, cyclohexyl or trifluoromethyl group.

3. A compound according to claim 1 or claim 2, wherein R₁ represents a methyl, methoxy or 2,2,2-trifluoroethoxy group.

4. A compound according to any preceding claim, wherein R₂ represents a hydrogen or fluorine atom.

5. A compound according to any preceding claim, wherein R₃ represents a hydrogen or chlorine atom or a 2,2,2-trifluoroethoxy group.

6. A compound according to any preceding claim, wherein n is 1.

7. Any one of the following compounds:
■ N-{3-[4-(5-chloro-2-methoxyphenyl)-1-piperazinyl]-propyl}-7-oxo-5-phenyl-7H-thieno[3,2-b]pyran-3-carboxamide,
■ N-{3-[4-(2-methoxyphenyl)-1-piperazinyl]-propyl}-7-oxo-5-phenyl-7H-thieno[3,2-b]pyran-3-carboxamide,
■ 5-cyclohexyl-N-{3-[4-(2-methoxyphenyl)-1-piperazinyl]-propyl}-7-oxo-7H-thieno[3,2-b]pyran-3-carboxamide,
■ N-{3-[4-(2-methoxyphenyl)-1-piperazinyl]-propyl}-7-oxo-5-trifluoromethyl-7H-thieno[3,2-b]pyran-3-carboxamide,
■ 7-oxo-5-phenyl-N-{3-[4-[2-(2,2,2-trifluoroethoxy)phenyl]- 1-piperazinyl]-propyl}-7H-thieno[3,2-b]pyran-3-carboxamide,
■ N-{3-[4-[2-methoxy-5-(2,2,2-trifluoroethoxy)-phenyl]-1-piperazinyl]-propyl}-7-oxo-5-phenyl-7H-thieno[3,2-b]pyran-3-carboxamide, and
■ N-{3-[4-[4-fluoro-2-(2,2,2-trifluoroethoxy)-phenyl]-1-piperazinyl]-propyl}-7-oxo-5-phenyl-7H-thieno[3,2-b]pyran-3-carboxamide.

8. A pharmaceutical composition comprising a compound according to any preceding claim or an N-oxide or a pharmaceutically acceptable salt of such a compound in admixture with a pharmaceutically acceptable diluent or carrier.

9. A pharmaceutical composition according to claim 8 further comprising an anticholinergic agent.

10. A pharmaceutical composition according to claim 9 in which the anticholinergic agent is one or more of tolterodine, oxybutinin, darifenacin, alvameline and temiverine.

## Patentansprüche

1. Verbindung der allgemeinen Formel I 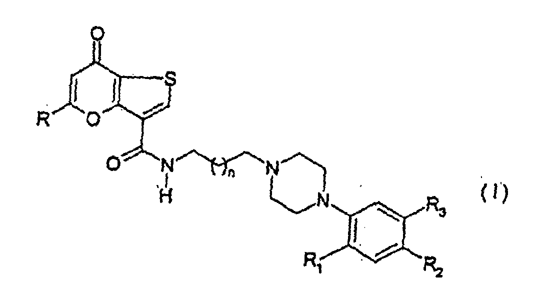 wobei R einen Aryl-, Cycloalkyl oder Polyhalogenalkylrest bedeutet,
R₁ einen Alkyl-, Alkoxy-, Polyfluoralkoxyrest, eine Hydroxy- oder Trifluormethansulfonyloxygruppe bedeutet,
R₂ und R₃ jeweils unabhängig voneinander ein Wasserstoff- oder Halogenatom oder einen Alkoxy- oder Polyfluoralkoxyrest bedeuten, und
n gleich 0, 1 oder 2 ist,
oder ein N-Oxid oder ein pharmazeutisch verträgliches Salz einer derartigen Verbindung.

2. Verbindung gemäß Anspruch 1, wobei R eine Phenyl-, Cyclohexyl- oder Trifluormethylgruppe bedeutet.

3. Verbindung gemäß Anspruch 1 oder 2, wobei R₁ eine Methyl-, Methoxy- oder 2,2,2-Trifluorethoxygruppe bedeutet.

4. Verbindung gemäß einem vorstehenden Anspruch, wobei R₂ ein Wasserstoff- oder Fluoratom bedeutet.

5. Verbindung gemäß einem vorstehenden Anspruch, wobei R₃ ein Wasserstoff- oder Chloratom oder eine 2,2,2-Trifluorethoxygruppe bedeutet.

6. Verbindung gemäß einem vorstehenden Anspruch, wobei n gleich 1 ist.

7. Eine der nachfolgenden Verbindungen:
- N-{3-[4-(5-Chlor-2-methoxyphenyl)-1-piperazinyl]propyl}-7-oxo-5-phenyl-7Hthieno[3,2-b]pyran-3-carboxamid,
- N-{3-[4-(2-Methoxyphenyl)-1-piperazinyl]propyl}-7-oxo-5-phenyl-7H-thieno [3,2-b]pyran-3-carboxamid,
- 5-Cyclohexyl-N-{3-[4-(2-methoxyphenyl)-1-piperazinyl]propyl}-7-oxo-7Hthieno[3,2-b]pyran-3 -carboxamid,
- N-{3-[4-(2-Methoxyphenyl)-1-piperazinyl]propyl}-7-oxo-5-trifluormethyl-7Hthieno[3,2-b]pyran-3-carboxamid,
- 7-Oxo-5-phenyl-N-{3-[4-[2-(2,2,2-trifluorethoxy)phenyl]-1-piperazinyl]propyl}-7H-thieno[3;2-b]pyran-3-carboxamid,
- N-{3-[4-[2-Methoxy-5-(2,2,2-trifluorethoxy)phenyl]-1-piperazinyl]propyl}-7-oxo-5-phenyl-7H-thieno[3,2-b]pyran-3-carboxamid, und
- N-{3-[4-[4-Fluor-2-(2,2,2-trifluorethoxy)phenyl]-1-piperazinyl]propyl}-7-oxo-5-phenyl-7H-thieno[3,2-b]pyran-3-carboxamid.

8. Arzneimittel, umfassend eine Verbindung gemäß einem vorstehenden Anspruch oder ein N-Oxid oder ein pharmazeutisch verträgliches Salz einer derartigen Verbindung in einer Beimischung mit einem pharmazeutisch verträglichen Verdünnungsmittel oder Träger.

9. Arzneimittel gemäß Anspruch 8, welches weiterhin ein Anticholinergikum umfasst.

10. Arzneimittel gemäß Anspruch 9, wobei das Anticholinergikum eines oder mehrere aus Tolterodin, Oxybutinin, Darifenacin, Alvamelin und Temiverin ist.

## Revendications

1. Composé ayant la formule générale I 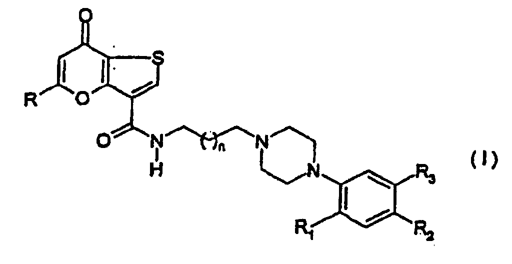 dans laquelle
R représente un groupe aryle, cycloalkyle ou polyhalogénoalkyle,
R₁ représente un groupe alkyle, alcoxy, polyfluoroalcoxy, hydroxy ou trifluorométhanesulfonyloxy,
chacun parmi R₂ et R₃ représente indépendamment un atome d'hydrogène ou d'halogène ou un groupe alcoxy ou polyfluoroalcoxy, et
n est 0, 1 ou 2,
ou un N-oxyde ou un sel pharmaceutiquement acceptable d'un tel composé.

2. Composé selon la revendication 1, dans lequel R représente un groupe phényle, cyclohexyle ou trifluorométhyle.

3. Composé selon la revendication 1 ou la revendication 2, dans lequel R₁ représente un groupe méthyle, méthoxy ou 2,2,2-trifluoroéthoxy.

4. Composé selon l'une quelconque des revendications précédentes, dans lequel R₂ représente un atome d'hydrogène ou de fluor.

5. Composé selon l'une quelconque des revendications précédentes, dans lequel R₃ représente un atome d'hydrogène ou de chlore ou un groupe 2,2,2-trifluoroéthoxy.

6. Composé selon l'une quelconque des revendications précédentes, dans lequel n est 1.

7. L'un quelconque des composés suivants :
■ N-{3-[4-(5-chloro-2-méthoxyphényl)-1-pipérazinyl]-propyl}-7-oxo-5-phényl-7H-thiéno[3,2-b]pyran-3-carboxamlde,
■ N-{3-[4-(2-méthoxyphényl)-1-pipérazinyl]-propyl}-7-oxo-5-phényl-7H-thiéno[3,2-b]pyran-3-carboxamide,
■ 5-cyclohexyl-N-{3-[4-(2-méthoxyphényl}-1-pipérazinyl]-propyl}-7-oxo-7H-thiéno[3,2-b]pyran-3-carboxamide,
■ N-{3-[4-(2-méthoxyphényl)-1-pipérazinyl]-propyl}-7-oxo-trifluorométhyl-7H-thiéno[3,2-b]pyran-3-carboxamide,
■ 7-oxo-5-phényl-N-{3-[4-[2-(2,2,2-trifluoroéthoxy)phényl]-1-pipérazinyl]-propyl}-7H-thiéno[3,2-b]pyran-3-carboxamide,
■ N-{3-[4-[2-méthoxy-5-(2,2,2-trifluoroéthoxy)-phényl]-1-pipérazinyl]-propyl}-7-oxo-5-phényl-7H-thiéno[3,2-b]pyran-3-carboxamide, et
■ N-{3-[4-[4-fluoro-2-(2,2,2-trifluoroéthoxy)-phényl]-1-pipérazinyl]-propyl}-7-oxo-5-phényl-7H-thiéno[3,2-b]pyran-3-carboxamide.

8. Composition pharmaceutique comprenant un composé selon l'une quelconque des revendications précédentes ou un N-oxyde ou un sel pharmaceutiquement acceptable d'un tel composé mélangé avec un diluant ou support pharmaceutiquement acceptable.

9. Composition pharmaceutique selon la revendication 8, comprenant en outre un agent anticholinergique.

10. Composition pharmaceutique selon la revendication 9, dans laquelle l'agent anticholinergique est une ou plusieurs substances parmi la toltérodine, l'oxybutinine, la darifénacine, l'alvaméline et la témivérine.
